# FASCICULE DE BREVET EUROPEEN

(11) **EP 3 160 435 B1**
(45) Date de publication et mention de la délivrance du brevet: **12.12.2018**
(21) Numéro de dépôt: 15732693.5
(22) Date de dépôt: 30.06.2015
(51) Int. Cl.: A61K 8/35, A61Q 19/00

(54) **UTILISATION DE 4-(3-ÉTHOXY-4-HYDROXYPHÉNYL)ALKYLCÉTONE EN TANT QU'AGENT APAISANT DE LA PEAU**
VERWENDUNG VON 4-(3-ETHOXY-4-HYDROXYPHENYL)ALKYLKETON ALS HAUTLINDERUNGSMITTEL
USE OF 4-(3-ETHOXY-4-HYDROXYPHENYL)ALKYLKETONE AS A SKIN-SOOTHING AGENT

(30) Priorité: 30.06.2014 FR 1456176
(43) Date de publication de la demande: 03.05.2017
(73) Titulaire: L'Oréal, 75008 Paris (FR)
(72) Inventeur: DALKO, Maria, F-78000 Versailles (FR)
(74) Mandataire: Kromer, Christophe
(86) Numéro de dépôt international: PCT/EP2015/064779
(87) Numéro de publication internationale: WO 2016/001189

(56) Documents cités:
- WO-A1-2011/039445
- WO-A1-2012/045809
- US-A1- 2012 258 058

## Description

La présente invention concerne l'utilisation cosmétique non thérapeutique de composés de type 4-(3-éthoxy-4-hydroxyphényl)alkylcétone, en tant qu'agents apaisants de la peau du visage et du corps.

La peau est la première barrière de protection de l'organisme vis-à-vis de l'environnement. Elle subit donc de nombreuses agressions extérieures qui peuvent conduire à des réactions cutanées d'inconfort.

Les réactions cutanées d'inconfort de la peau du visage ou du corps peuvent notamment être induites par le contact avec des produits chimiques tels que les nettoyants, les permanentes, les colorations du cheveu ou provenir d'actions mécaniques tels que le rasage, le gommage, le peeling, l'épilation, ou provenir de l'action de la température, du climat, ou encore de la pollution atmosphérique.

Il existe donc un besoin pour de nouveaux agents apaisants dans le domaine cosmétique pour la peau du visage et du corps.

Il est décrit dans la demande WO2012/131274 que des composés de type 4-(3-éthoxy-4-hydroxyphényl)alkylcétone permettent d'éliminer et/ou réduire le nombre de levure du genre Malassezia, le nombre de pellicules, ainsi que les démangeaisons et les rougeurs sur le cuir chevelu.

Toutefois la peau du visage ou du corps ne subit pas les mêmes désagréments du scalp induit par la présence en quantité importante de la levure du genre Malassezia. US2012258058 concerne une formulation ayant une action de réduction de l'irritation de la peau en utilisant du [6]-paradol dans une composition cosmétique.

De façon surprenante, l'inventeur a montré que les composés de formule (I) décrits ci-après présentent des propriétés apaisantes pour la peau du visage et/ou du corps..

La présente invention concerne l'utilisation cosmétique non thérapeutique en tant qu'agent apaisant pour la peau du visage et/ou du corps d'un composé de formule (I) suivante : 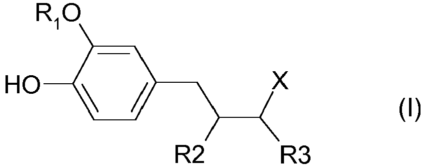 dans laquelle :
- R1 représente un radical alkyle en C1-C4
- R2 représente un atome d'hydrogène, ou un radical hydrocarboné, saturé ou insaturé, linéaire ou ramifié, en C1-C6 ;
- R3 représente un radical hydrocarboné, saturé ou insaturé, linéaire ou ramifié, en C1-C4 éventuellement substitué par un groupe hydroxyle ;
- C-X représente C=O ou CH-OH.

De préférence :
- R1 représente un radical alkyle éthyle
- R2 représente un atome d'hydrogène ;
- R3 représente un radical hydrocarboné, saturé ou insaturé, linéaire ou ramifié, en C1-C4 éventuellement substitué par un groupe hydroxyle ;
- C-X représente C=O ou CH-OH et de préférence C=O.

Préférentiellement :
- R1 représente un radical alkyle éthyle
- R2 représente un atome d'hydrogène ;
- R3 représente un radical hydrocarboné, saturé, linéaire en C1-C3 ;
- C-X représente C=O.

Comme exemple de composés (I) on peut citer :

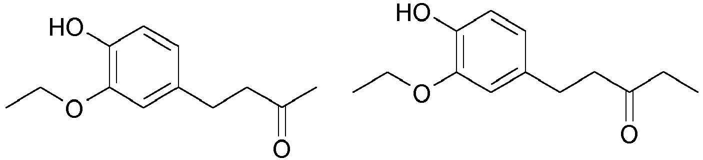

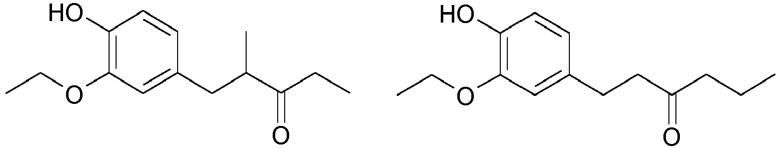

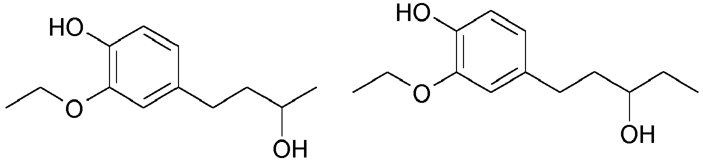

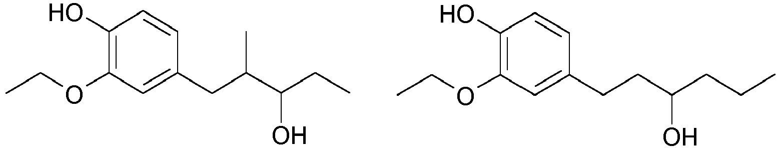

De préférence, on utilise le composé :

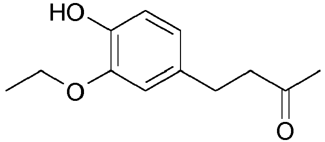

Les composés de formule (I) susmentionnée peuvent donc être utilisés dans des compositions cosmétiques.

On entend par « composition cosmétique » une substance ou une préparation destinée à être mise en contact avec les diverses parties superficielles du corps humain, notamment l'épiderme, les ongles, les lèvres en vue, exclusivement ou principalement, de les nettoyer, de les parfumer, d'en modifier l'aspect, de les protéger, de les maintenir en bon état ou de corriger les odeurs corporelles. Une composition cosmétique est destinée à une utilisation non thérapeutique.

La composition cosmétique est destinée à être appliquée sur la peau.

Par « peau », on entend la peau du visage et/ou du corps (cou, mains, pieds, jambes, buste).

L'utilisation cosmétique des composés de formule (I) susmentionnée selon l'invention peut être destinée à prévenir et/ou traiter les réactions inesthétiques et/ou d'inconfort de la peau, ces réactions étant d'origine non pathologique et pouvant être occasionnées, par exemple, par le rasage ou l'épilation.

En particulier, l'utilisation cosmétique selon l'invention peut être destinée à prévenir et/ou traiter au moins une réaction cutanée de la peau, cette réaction étant d'origine non pathologique, choisie dans le groupe constitué des rougeurs, des sensations de picotements ou de tiraillements.

De manière générale, les réactions cutanées non pathologiques évoquées précédemment sont plus fréquentes dans les zones les plus exposées de l'organisme, à savoir les mains, les pieds, les jambes, le visage et le cou. Elles peuvent survenir, notamment, sur des zones soumises à certains gestes d'hygiène quotidienne, ou fréquemment renouvelés, tels que le rasage (barbe, jambes), l'épilation, la détersion par des produits de toilette ou des produits ménagers, l'application d'adhésifs comme des pansements, des patchs, ou des fixations de prothèses, ou dans le cas de gestes sportifs ou professionnels, ou simplement liés au mode de vie, à l'utilisation de vêtements, d'outils ou d'équipements générant des frottements localisés, ou à l'exposition à des agents irritants ou polluants ou aux conditions climatiques.

Les compositions cosmétiques comprenant des composés de formule (I), tels que définis ci-dessus comprennent en outre un milieu physiologiquement acceptable. Un milieu physiologiquement acceptable est un milieu non toxique et susceptible d'être appliqué sur la peau et les phanères des êtres humains et d'aspect, d'odeur et de toucher agréables.

Le composé apaisant de formule (I) tel que défini ci-dessus est de préférence présent dans les compositions cosmétiques en une quantité de 0,01% à 10% en poids par rapport au poids total de la composition.

Préférentiellement, le composé apaisant de formule (I) tel que défini ci-dessus est présent en une quantité de 0,1% à 5 % en poids par rapport au poids total de la composition.

Le composé apaisant de formule (I) tel que défini ci-dessus est encore préférentiellement présent en une quantité de 0,1% à 3% en poids par rapport au poids total de la composition.

La composition cosmétique peut comprendre en outre en tant qu'excipient au moins un gélifiant hydrophile ou lipophile, un additif hydrophile ou lipophile, un agent émulsifiant, un agent stabilisant, un conservateur, une charge, un parfum, un absorbeur d'odeurs, une huile, une cire, un solvant ou une matière colorante.

Les quantités de ces différents excipients sont celles classiquement utilisées dans le domaine cosmétique, et par exemple varient d'environ 0,01 % à 15 % du poids total de la composition.

Selon un mode particulier de la présente invention, la composition cosmétique est destinée à une administration topique.

Pour une application topique sur la peau, la composition peut avoir la forme notamment de solutions aqueuses ou huileuses ou de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique ou de mousses, de préférence sous la forme d'émulsion. Ces compositions sont préparées selon les méthodes usuelles.

Ces compositions cosmétiques ou dermatologiques peuvent par exemple constituer des crèmes de nettoyage, de protection, de traitement ou de soin pour le visage, pour les mains, pour les pieds, pour les grands plis anatomiques ou pour le corps (par exemple crèmes de jour, crèmes de nuit, crèmes de démaquillage, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage et des crèmes épilatoires.

Les compositions peuvent également consister en des préparations solides constituant des savons ou des pains de nettoyage.

Les compositions peuvent aussi être conditionnées sous forme de composition pour aérosol comprenant également un agent propulseur sous pression.

La composition peut être formulée sous forme d'émulsion, de crème, de pommade, de baume, de lait, de lotion, de gel, de mousse ou de solution.

La composition cosmétique peut en particulier être choisie parmi les produits de rasage, les produits après-soleil, les déodorants, les crèmes apaisantes, ou les produits spécifiquement destinés aux lèvres, aux yeux ou aux jambes.

En particulier, la composition cosmétique peut être choisie parmi les baumes après-rasage, les déodorants ou les crèmes apaisantes.

L'invention a aussi pour objet un procédé cosmétique pour le soin non thérapeutique pour apaiser la peau du visage et/ou du corps, caractérisé en ce qu'il comprend l'application sur la peau du visage et/ou du corps, d'une composition cosmétique telle que décrite précédemment. Le procédé est mis en oeuvre sur la peau du visage et/ou du corps, à l'exclusion du scalp.

Le procédé cosmétique selon l'invention est destiné à apaiser la peau, en particulier pour prévenir et/ou réduire les réactions d'inconfort de la peau du visage et/ou du corps.

Plus particulièrement, le procédé cosmétique selon l'invention est destiné à prévenir et/ou traiter au moins une réaction cutanée choisie dans le groupe constitué des rougeurs, des sensations de picotements ou de tiraillements. Il s'agit en particulier de sensations de picotements ou de tiraillements dûs à la sécheresse de la peau.

Les propriétés des composés apaisants de formule (I) tels que définis précédemment ont été mises en évidence grâce notamment à l'exemple ci-dessous.

### Exemple 1 :

On prépare un gel cosmétique ayant la composition suivante :
- composé 0,1 % 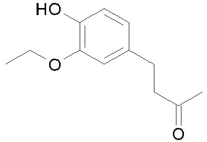
- acide acrylique réticulé (CARBOPOL 941)0,3 %
- eau qsp 100 %

Le gel appliqué sur une peau du visage préalablement rasée ou sur les jambes préalablement épilées permet d'apaiser la peau suite au rasage ou à l'épilation.

### Exemple 2 :

On a évalué les propriétés apaisantes du composé : 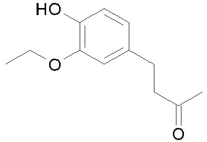 avec le protocole suivant :
On a préparé la composition suivante (Composition A) :
Composé

| | | |
|---|---|---|
| 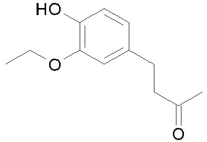 | | 1,6 g |
| Homopolymère AMPS | | 0,19 g |
| Cyclohexasiloxane | | 5 g |
| Dimethicone 10 cst | | 3,75 g |
| Polyisobutylène hydrogéné (Parleam de chez Nof Corporation) | | 4 g |
| Hydroxyde de sodium | | 0,12 g |
| Propane 1-3 diol | | 5 g |
| Polymère carboxyvinylique (Carbopol 980 polymer de chez Lubrizol) | | 0,3 g |
| Copolymère réticulé acide acrylique/methacrylate d'alkyl C₁₀-C₃₀ (PEMULEN TR-2 POLYMER de chez Lubrizol) | | 0,11 g |
| Acide poly acrylamidométhyl propane sulfonique neutralisé partiellement à l'ammoniaque et hautement réticulé (Hostacerin AMPS® de chez Clariant) | | 0,2 |
| Acide citrique | | 0,08 g |
| Eau | qsp | 100 g |

On a également préparé une composition placébo (composition P) sans le composé cétonique en remplaçant sa quantité par de l'eau .

On a effectué un test panel avec 10 hommes pour évaluer la composition A et avec 9 hommes pour évaluer la composition P. Les hommes du panel ont une peau blanche.

Chaque sujet a appliqué la composition après le rasage le matin et également le soir avant le coucher, pendant 5 jours.
Une première évaluation ou mesure a été effectuée le premier jour 20 minutes après le rasage, puis une deuxième évaluation ou mesure a été faite le 5^{ème} jour 20 minutes après le rasage, comme décrit ci-après.

Mesure de la rougeur de la peau :
On a mesuré la rougeur de la peau avec un chromamètre (Konica Minolta CR-400 , D65 Daylight mode) sur la zone du visage traitée avec la composition et la zone non traitée. La couleur a été mesurée en utilisant le système CIE L a b et la valeur a a été notée (3 mesures effectuées).
Avec la composition A on a obtenu une réduction de la rougeur de 4,1 % tandis qu'avec la composition placébo la réduction de la rougeur est de 1,4 %.

Evaluation de la sensation de tiraillement de sécheresse de la peau :
On a également demandé au panel d'évaluer la sensation de tiraillement de sécheresse de la peau au premier jour et au cinquième jour de traitement selon la notation suivante :
0 = aucune
1 = faible
2 = modérée
3 = sévère

On a ensuite calculé la moyenne de la note attribuée par l'ensemble du panel au premier et au cinquième jour de traitement puis déterminée l'écart en pourcentage entre la
On a obtenu les résultats suivants :
Composition A :
   Moyenne premier jour = 0,90
   Moyenne cinquième jour = 0,30
   Soit une différence de 0,3/0,9 x 100 = 33, 3 %
Composition P :
   Moyenne premier jour = 0,78
   Moyenne cinquième jour = 0,44
   Soit une différence de 0,44/0,78 x 100 = 57, 1 %

Le panel traité avec la composition A perçoit une sensation de tiraillement de sécheresse de la peau plus faible que celle perçu par le panel traité avec la composition P.

Les résultats obtenus montrent que le composé cétonique testé permet de diminuer la rougeur de la peau ainsi que la sensation de tiraillement de sécheresse de la peau occasionnée par le rasage. Le composé cétonique a donc une action apaisante de la peau après le rasage.

## Revendications

1. Utilisation cosmétique non thérapeutique d'un composé de formule (I) suivante : 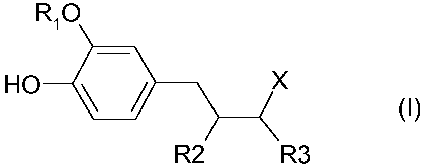 dans laquelle :
- R1 représente un radical alkyle en C1-C4
- R2 représente un atome d'hydrogène, ou un radical hydrocarboné, saturé ou insaturé, linéaire ou ramifié, en C1-C6 ;
- R3 représente un radical hydrocarboné, saturé ou insaturé, linéaire ou ramifié, en C1-C4 éventuellement substitué par un groupe hydroxyle ;
- C-X représente C=O ou CH-OH ;
en tant qu'agent apaisant de la peau du visage ou du corps.

2. Utilisation cosmétique selon la revendication 1, dans laquelle pour le composé de formule (I) :
- R1 représente un radical alkyle éthyle
- R2 représente un atome d'hydrogène ;
- R3 représente un radical hydrocarboné, saturé ou insaturé, linéaire ou ramifié, en C1-C4 éventuellement substitué par un groupe hydroxyle ;
- C-X représente C=O ou CH-OH .

3. Utilisation cosmétique selon la revendication 1 ou 2, dans laquelle pour le composé de formule (I) :
- R1 représente un radical alkyle éthyle
- R2 représente un atome d'hydrogène ;
- R3 représente un radical hydrocarboné, saturé, linéaire en C1-C3 ;
- C-X représente C=O.

4. Utilisation cosmétique selon la revendication 1, dans laquelle le composé de formule (I) est choisi parmi :

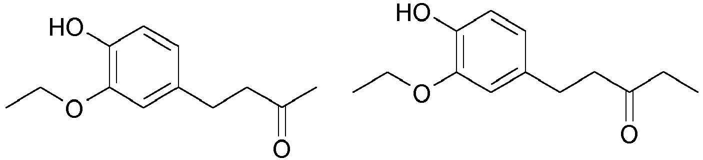

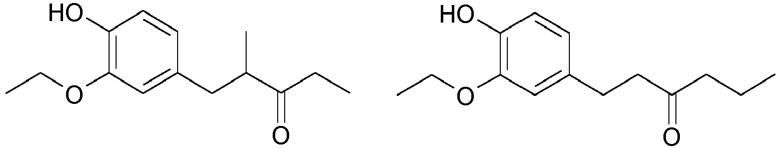

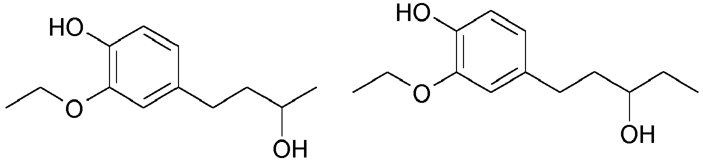

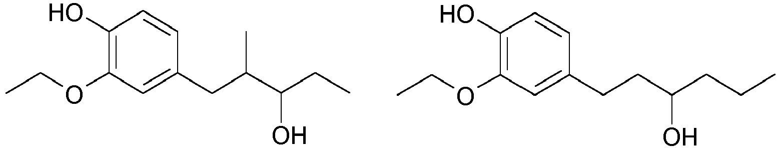

5. Utilisation cosmétique selon l'une quelconque des revendications précédentes, dans laquelle le composé (I) est :

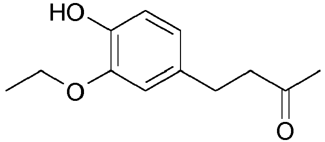

6. Utilisation cosmétique selon l'une quelconque des revendications précédentes, pour prévenir et/ou traiter les réactions d'origine non pathologiques inesthétiques et/ou d'inconfort de la peau.

7. Utilisation cosmétique selon l'une quelconque des revendications précédentes, pour prévenir et/ou traiter au moins une réaction cutanée non pathologique choisie dans le groupe constitué des rougeurs, des sensations de picotements ou de tiraillements.

8. Utilisation cosmétique selon la revendication précédente, dans laquelle les sensations de picotement ou de tiraillement sont dûs à la sécheresse de la peau.

9. Utilisation cosmétique selon l'une quelconque des revendications 1 à 8, dans laquelle le composé de formule (I) est compris dans une composition cosmétique.

10. Utilisation cosmétique, dans laquelle le composé apaisant de formule (I) tel que défini à l'une quelconque des revendications 1 à 8 est présent dans une composition cosmétique en une quantité allant de 0,01% à 10% en poids par rapport au poids total de la composition, de préférence allant de 0,1 % à 5 % en poids.

11. Procédé cosmétique non thérapeutique pour apaiser la peau du visage et/ou du corps, **caractérisé en ce qu'**il comprend l'application sur la peau du visage et/ou du corps, d'une composition cosmétique comprenant un composé (I) défini selon l'une des revendications 1 à 5.

12. Procédé selon la revendication précédente, pour prévenir et/ou traiter les réactions d'origine non pathologiques inesthétiques et/ou d'inconfort de la peau.

13. Procédé selon l'une quelconque des revendications 11 ou 12, pour prévenir et/ou traiter au moins une réaction cutanée non pathologique choisie dans le groupe constitué des rougeurs, des sensations de picotements ou de tiraillements.

14. Procédé selon l'une quelconque des revendications 11 à 13, dans lequel le composé de formule (I) est compris dans une composition cosmétique.

15. Procédé selon l'une quelconque des revendications 11 à 13, dans lequel le composé de formule (I) est présent dans une composition cosmétique en une quantité allant de 0,01% à 10% en poids, par rapport au poids total de la composition, de préférence allant de 0,1 % à 5 % en poids.

## Patentansprüche

1. Nicht-therapeutische kosmetische Verwendung einer Verbindung der folgenden Formel (I): 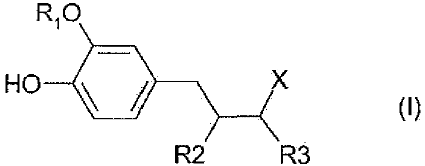 wobei:
- R1 für einen C1-C4-Alkylrest steht
- R2 für ein Wasserstoffatom oder einen gesättigten oder ungesättigten, geradkettigen oder verzweigten C1-C6-Kohlenwasserstoffrest steht;
- R3 für einen gesättigten oder ungesättigten, geradkettigen oder verzweigten C1-C4-Kohlenwasserstoffrest steht, der möglicherweise mit einer Hydroxylgruppe substituiert ist;
- C-X für C=O oder CH-OH steht;
als Mittel zur Beruhigung der Gesichts- oder Körperhaut.

2. Kosmetische Verwendung nach Anspruch 1, wobei für die Verbindung nach Formel (I):
- R1 für einem Ethylalkylrest steht
- R2 für ein Wasserstoffatom steht;
- R3 für einen gesättigten oder ungesättigten, geradkettigen oder verzweigten C1-C4-Kohlenwasserstoffrest steht, der möglicherweise mit einer Hydroxylgruppe substituiert ist;
- C-X für C=O oder CH-OH steht.

3. Kosmetische Verwendung nach Anspruch 1 oder 2, wobei für die Verbindung nach Formel (I):
- R1 für einem Ethylalkylrest steht
- R2 für ein Wasserstoffatom steht;
- R3 für einen gesättigten, geradkettigen C1-C3-Kohlenwasserstoffrest steht;
- C-X für C=O steht.

4. Kosmetische Verwendung nach Anspruch 1, wobei die Verbindung nach Formel (I) aus den folgenden ausgewählt ist:

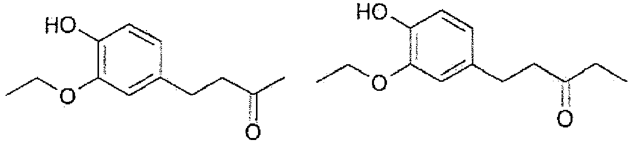

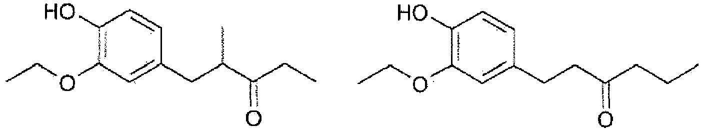

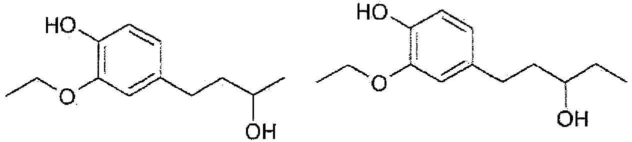

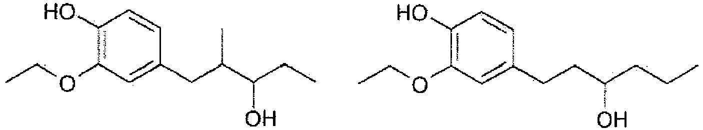

5. Kosmetische Verwendung nach einem beliebigen der vorhergehenden Ansprüche, wobei es sich bei der Verbindung (I) um die folgende handelt:

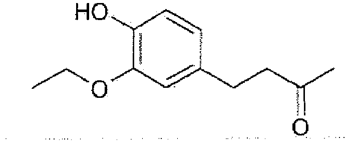

6. Kosmetische Verwendung nach einem beliebigen der vorhergehenden Ansprüche, um Hautreaktionen zu verhüten und/oder behandeln, die unästhetisch und nicht-krankheitsbedingten Ursprungs sind und/oder mit Unwohlsein einhergehen.

7. Kosmetische Verwendung nach einem beliebigen der vorhergehenden Ansprüche, um mindestens eine nichtkrankheitsbedingte Hautreaktion zu verhüten und/oder zu behandeln, die aus der Gruppe ausgewählt ist, welche aus Rötungen, Kribbelgefühl oder Spannungen besteht.

8. Kosmetische Verwendung nach dem vorhergehenden Anspruch, wobei das Kribbelgefühl oder die Spannungen auf Hauttrockenheit zurückzuführen sind.

9. Kosmetische Verwendung nach einem beliebigen der Ansprüche 1 bis 8, wobei die Verbindung nach Formel (I) Bestandteil einer kosmetischen Zusammensetzung ist.

10. Kosmetische Verwendung, wobei die beruhigende Verbindung der Formel (I) gemäß der Begriffsbestimmung in einem beliebigen der Ansprüche 1 bis 8 in einer kosmetischen Zusammensetzung in einer Menge vorliegt, die im Bereich von 0,01 % bis 10 % nach Gewicht liegt, bezogen auf das Gesamtgewicht der Zusammensetzung, vorzugsweise im Bereich von 0,1 % bis 5 % nach Gewicht.

11. Nicht-therapeutisches kosmetisches Verfahren, um die Gesichts- und/oder Körperhaut zu beruhigen, **dadurch gekennzeichnet, dass** es das Aufbringen einer kosmetischen Zusammensetzung, die eine Verbindung (I) gemäß der Begriffsbestimmung in einem der Ansprüche 1 bis 5 umfasst, auf die Gesichts- und/oder Körperhaut umfasst.

12. Verfahren nach dem vorhergehenden Anspruch, um Hautreaktionen zu verhüten und/oder behandeln, die unästhetisch und nicht-krankheitsbedingten Ursprungs sind und/oder mit Unwohlsein einhergehen.

13. Verfahren nach einem beliebigen der Ansprüche 11 oder 12, um mindestens eine nichtkrankheitsbedingte Hautreaktion zu verhüten und/oder zu behandeln, die aus der Gruppe ausgewählt ist, welche aus Rötungen, Kribbelgefühl oder Spannungen besteht.

14. Verfahren nach einem beliebigen der Ansprüche 11 bis 13, wobei die Verbindung nach Formel (I) Bestandteil einer kosmetischen Zusammensetzung ist.

15. Verfahren nach einem beliebigen der Ansprüche 11 bis 13, wobei die Verbindung der Formel (I) in einer kosmetischen Zusammensetzung in einer Menge vorliegt, die im Bereich von 0,01 % bis 10 % nach Gewicht liegt, bezogen auf das Gesamtgewicht der Zusammensetzung, vorzugsweise im Bereich von 0,1 % bis 5 % nach Gewicht.

## Claims

1. Non-therapeutic cosmetic use of a compound of formula (I) below: 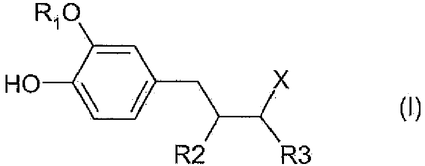 in which:
- R1 represents a C1-C4 alkyl radical ;
- R2 represents a hydrogen atom, or a saturated or unsaturated, linear or branched C1-C6 hydrocarbon-based radical;
- R3 represents a saturated or unsaturated, linear or branched C1-C4 hydrocarbon-based radical optionally substituted by a hydroxyl group;
- C-X represents C=O or CH-OH;
as agent for soothing the skin of the face or body.

2. Cosmetic use according to Claim 1, in which, for the compound of formula (I):
- R1 represents an ethyl alkyl radical;
- R2 represents a hydrogen atom;
- R3 represents a saturated or unsaturated, linear or branched C1-C4 hydrocarbon-based radical optionally substituted by a hydroxyl group;
- C-X represents C=O or CH-OH.

3. Cosmetic use according to Claim 1 or 2, in which, for the compound of formula (I):
- R1 represents an ethyl alkyl radical;
- R2 represents a hydrogen atom;
- R3 represents a saturated linear C1-C3 hydrocarbon-based radical;
- C-X represents C=O.

4. Cosmetic use according to Claim 1, in which the compound of formula (I) is chosen from:

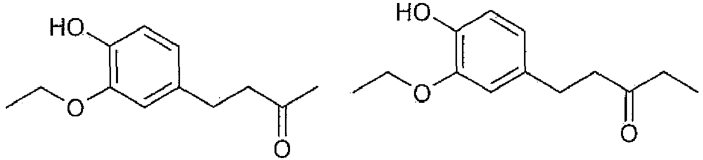

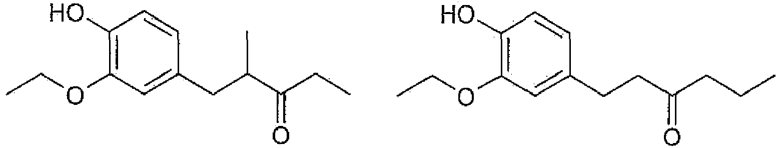

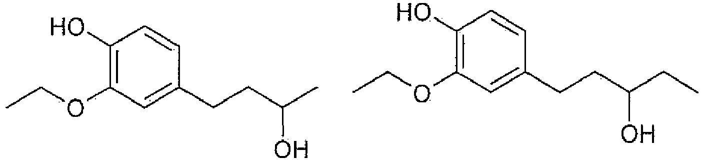

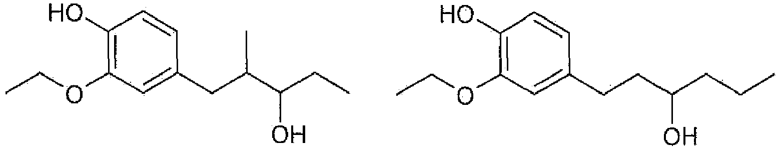

5. Cosmetic use according to any one of the preceding claims, in which the compound (I) is:

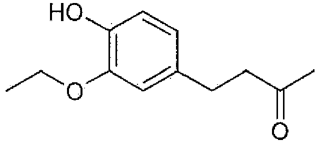

6. Cosmetic use according to any one of the preceding claims, for preventing and/or treating unattractive non-pathological reactions and/or skin discomfort.

7. Cosmetic use according to any one of the preceding claims, for preventing and/or treating at least one non-pathological skin reaction chosen from the group consisting of redness, stinging or tautness sensations.

8. Cosmetic use according to the preceding claim, wherein the stinging or tautness sensations are due to skin dryness.

9. Cosmetic use according to any one of Claims 1 to 8, wherein the compound of formula (I) is contained in a cosmetic composition.

10. Cosmetic use, wherein the soothing compound of formula (I) as defined in any one of Claims 1 to 8 is present in a cosmetic composition in an amount ranging from 0.01% to 10% by weight, preferably ranging from 0.1% to 5% by weight relative to the total weight of the composition.

11. Non-therapeutic cosmetic process for soothing the skin of the face and/or body, **characterized in that** it comprises the application, to the skin of the face and/or body, of a cosmetic composition comprising a compound (I) defined in one of Claims 1 to 5.

12. Process according to the preceding claim, for preventing and/or treating unattractive non-pathological reactions and/or skin discomfort.

13. Process according to either one of Claims 11 and 12, for preventing and/or treating at least one non-pathological skin reaction chosen from the group consisting of redness, stinging or tautness sensations.

14. Process according to any one of Claims 11 to 13, wherein the compound of formula (I) is contained in a cosmetic composition.

15. Process according to any one of Claims 11 to 13, wherein the compound of formula (I) is present in a cosmetic composition in an amount ranging from 0.01% to 10% by weight and preferably ranging from 0.1% to 5% by weight relative to the total weight of the composition.
